(19) Europäisches Patentamt
European Patent Office
Office européen des brevets

(11) Numéro de publication: **0 097 584**
**B1**

# (12) FASCICULE DE BREVET EUROPEEN

(45) Date de publication du fascicule du brevet: **09.04.86**

(21) Numéro de dépôt: **83401241.1**

(22) Date de dépôt: **16.06.83**

(51) Int. Cl.[4]: **C 07 D 409/04**, A 61 K 31/395, A 61 K 31/44, A 61 K 31/47, A 61 K 31/50 // (C07D409/04, 333:48, 213:59, 215:12, 335:02)

(54) **Nouveaux dérivés du thioformamide, leur préparation et les médicaments qui les contiennent.**

(30) Priorité: **17.06.82 FR 8210614**

(43) Date de publication de la demande: **04.01.84 Bulletin 84/1**

(45) Mention de la délivrance du brevet: **09.04.86 Bulletin 86/15**

(84) Etats contractants désignés: **AT BE CH DE FR GB IT LI LU NL SE**

(56) Documents cités:
**EP-A-0 046 417**
**GB-A-2 046 265**

**Le dossier contient des informations techniques présentées postérieurement au dépôt de la demande et ne figurant pas dans le présent fascicule.**

(73) Titulaire: **RHONE-POULENC SANTE, Les Miroirs 18 Avenue d'Alsace, F-92400 Courbevoie Cedex (FR)**

(72) Inventeur: **Aloup, Jean-Claude, 53, rue Marcel Risser, F-94290 Villeneuve-le-Roi (FR)**
Inventeur: **Bouchaudon, Jean, 23, avenue des Saules, F-91390 Morsang-sur-Orge (FR)**
Inventeur: **Farge, Daniel, 30, rue des Pins Sylvestres, F-94320 Thiais (FR)**
Inventeur: **James, Claude, 31 bis, avenue Gambetta, F-75020 Paris (FR)**

(74) Mandataire: **Le Goff, Yves et al, RHONE-POULENC RECHERCHES Brevets Pharma 25, Quai Paul Doumer, F-92408 Courbevoie (FR)**

**EP 0 097 584 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

La présente invention concerne de nouveaux dérivés du thioformamide de formule générale:

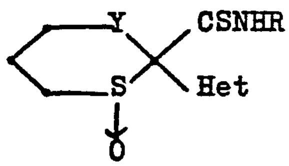

(I)

leur préparation et les médicaments qui les contiennent.

Dans la formule générale (I), R représente un atome d'hydrogène ou un radical alcoyle contenant 1 à 4 (de préférence 1 ou 2) atomes de carbone en chaîne droite ou ramifiée, Het représente un radical hétérocyclique à caractère aromatique contenant un atome d'azote choisi-parmi pyridyle-3 et quinolyle-3, et Y représent une liaison d vaéenc ou un radica méthylène.

La présence d'un atome d'oxygène sur le soufre crée dans la molécule une dissymétrie qui, associée au carbone asymétrique voisin, conduit à 4 stéréoisomères pouvant être éventuellement séparés en deux couples racémiques désignés ci-après par "Forme A", ou "produit le plus polaire" et "Forme B", ou "produit le moins polaire" [la polarité étant déterminée par chromatographie sur couche mince (C.C.M.)]. Ces formes peuvent elles-mêmes être dédoublées. Il est entendu que la présente invention concerne toutes les formes stéréoisomères ainsi que leurs mélanges.

Selon l'invention, les produits de formule générale (I) peuvent être préparés par action d'une amine de formule générale:

$R - NH_2$ (II)

dans laquelle R est défini comme précédemment, sur un dithioester de formule générale:

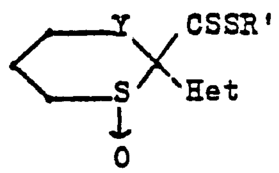

(III)

dans laquelle las symboles Het et Y sont définis comme précédemment et R' représente un radical alcoyle contenant 1 à 4 atomes de carbone en chaîne droite ou ramifiée ou un radical benzyle ou carboxyméthyle, suivie, le cas échéant, d'une séparation des formes A et B obtenues.

Généralement, on opère avec un excès d'amine de formule générale (II), sans solvant ou dans un solvant organique tel qu'un carbure aromatique, un éther ou un alcool à bas poids moléculaire ou un mélange de ces solvants, à une température comprise entre 20 et 130°C, éventuellement sous pression.

Il est particulièrement avantageux de fixer le thiol formé au cours de la réaction sous forme d'un sel de métal lourd en utilisaut un accepteur da thiol tel que le chlorure mercurique.

La séparation des formes A et B peut se faire par cristallisation ou par chromatographie du mélange obtenu.

Les dithioesters sulfoxydes de formule générale (III) peuvent être obtenus selon les méthodes suivantes:

1) par action d'une base forte sur un produit de formule générale:

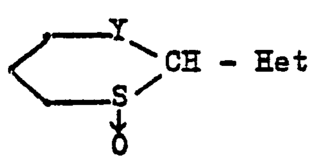

(IV)

dans laquelle Het et Y sont définis comme précédemment, suivis de l'action du sulfure de carbone puis d'un produit de formule générale:

R' - Z (V)

dans laquelle R' est défini comme précédemment et Z représents un atoms d'halogène, ds préférence un atome de chlore, de brome ou d'iods ou un reste d'ester réactif, ds préférence un reste mésyloxy ou tosyloxy.

La réaction s'effectue généralement dans un éther tel que le tétrahydrofuranne, généralement additionné

d'hexaméthyl, phosphoramide, à une température comprise entre −20 et +50°C.

Comme base forte, il est particulièrement avantageux d'employer le tert-butylate de potassium.

Les produits de formule générale (III) ainsi obtenus se présentent sous forme d'un mélange de sulfoxydes de forms stéréochimiques différentes dont le pourcentage relatif varie selon la nature de Y: lorsque Y représente une liaison de valence, le sulfoxyde de formule (III) existe essentiellement sous la forme qui, traitée par l'amine de formule générale (II) conduit au thioamide de formule générale (I) de forme B (la moins polaire); lorsque Y représente un radical méthylène, le sulfoxyde de formule (III) existe essentiellement sous la forme qui, traitée par l'amine de formule générale (II) conduit au thioamide de formule générale (I) de forme A (la plus polaire).

Les produits de formule générale (IV) peuvent être préparés selon l'une des méthodes suivantes:

a) - Par cyclisation d'un produit de formule générale:

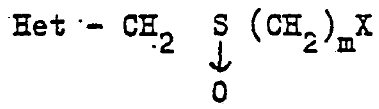

(VI)

dans laquelle Het est défini comme précédemment, m représente un nombre entier égal à 3 ou 4 et X représente un atome d'halogène, de préférence de chlore ou de brome, ou un reste d'ester réactif, de préférence un reste mésyloxy ou tosyloxy.

On opère généralement dans un solvant organique anhydre tel que le tétrahydrofuranne ou l'hexaméthylphosphoramide ou un mélange de ces solvants, à une température comprise entre -20 et +50°C en présence d'une base organique telle que le tert-butylate de potassium.

Dans la pratique, il est possible de préparer la dithioester de formule générale (III) à partir du produit de formule générale (VI) sans isoler le produit de formule générale (IV). Dans ce cas, on cyclise le produit de formule générale (VI) dans les conditions indiquées précédemment, en employant au moins deux équivalents de tert-butylate de potassium, puis on ajoute au mélange réactionnel le sulfure de carbone et le produit de formule générale (V) en opérant comme indiqué précédemment.

Les produits de formule générale (VI) peuvent être obtenus par oxydation d'un suifure de formule générale:

Het - $CH_2S\ (CH_2)_mX$ (VII)

dans laquelle Het est défiui comme précédemment, m représente un nombre entier égal à 5 ou 4 et X représente un atome d'halogène, de préférence de chlore ou de brome, ou un rester d'ester reactif, de préference un reste mésyloxy ou tosyloxy.

On realise l'oxydation en employant un équivalent d'un agent couramment utilisé pour passer d'un sulfure à un sulfoxyde, en opérant dans un solvant approprié. Par exemple, on peut employer l'eau oxygénée dans l'acétone ou dans l'acide acétique, un périodate alcalin dans un solvant hydroorganique tel que eau-éthanol ou eau-acétonitrile, un peroxyacide carboxylique (acide peracétique, perbenzoïque, m-chloroperbenzoïque, p-nitroperbenzoique ou perphtalique) dans un solvant chloré (dichlorométhane, dichloroéthene), dans l'acide acétique ou dans un mélange de ces solvants. La réaction s'effectue généralement à une température comprise entre −10 et +30°C.

Dans la pratique, il est particulièrement avantageux d'utiliser l'acide m-chloroperbenzoïque en opérant dans le cblorure de méthylène à une température voisine de 20°C.

Les sulfures de formule générale (VII) peuveut être obtenus selon la methode decrite dans la demande de brevst européen publiée sous le numéro 0046.417.

b) - par oxydation d'un produit de formule générale:

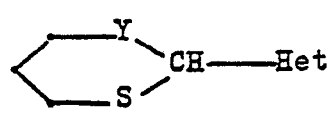

(VIII)

dans laquelle Y at Het sont définis comme précédemment.

L'oxydation est réalisea dans las conditions exposées précédemment pour la préparation das produits de formule générale (VI).

Les produits de formule générale (VIII) peuvent être preparés selon la méthode décrite dans la demande de brevat européen sous le numéro 0046.417.

2) Par oxydation d'un dithioester de formule générale:

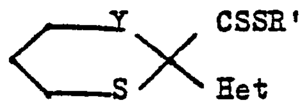

(IX)

dans laquelle les symboles Het, Y et R' sont définis comme précédemment.

L'oxydation des produits de formule générale (IX) peut être réalisée dans les conditions exposées précédemment pour la préparation des produits de formule générale (VI).

Les produits de formule générale (III) ainsi obtenus selon le procédé 2 se présentent sous forme d'un mélange de sulfoxydes diastéréoisomères dont les pourcentages relatifs varient entre 50-50 et 75-25 selon la nature des symboles Y, R' et Het.

Les produits de formule générale (III) obtenus selon les procédés 1 ou 2 décrits ci-dessus peuvent être employés,soit directement sous forme de mélangs des formes diastéréoisomères, soit après séparation de ces formes,pour préparer les produits de formule générale (I). La séparation des formes diastéréoisomères de ces produits peut être effectuée par cristallisation fractionnée ou de préference par chromatographie.

Les dithioesters de formule générale (IX) peuvent être préparés selon la méthode décrite dans la demande de brevet européen publiée sous le numéro 0046.417.

Les nouveaux produits selon l'invention peuvent être purifiés par les méthodes physiques habituelles, notamment la cristallisation et la chromatographie.

On connait déjà par la demande de brevet européen publiée sous le numéro 0046.417 les sulfures correspondant aux produits de la présente invention qui sont actifs comme antihypertenseurs.

Les nouveaux produits selon l'invention présentent des propriétés anti-hypertensives améliorées que ne laissait pas p[illegible]'art antérieur.

A des doses comprises entre 0,02 et 50 mg/kg p.o., ils abaissent la pression artérielle chez le rat spontanément hypertendu (rat SHR) de souche OKAMOTO-AOKI. L'utilisation du rat spontanément hypertendu pour l'étude des produits anti-hypertenseurs est décrite par J.L. ROBA, Lab. Anim. Sci., 26, 505 (1976).

Leur dose létale ($DL_{50}$)chez la souris, est généralement supérieure à 300 mg/kg p.o..

Comme il a été dit précédemment, la presence d'un atome d'oxygène sur l'atome de soufre crée dans la molécule une dissymétrie qui, associée au carbone asymétrique voisin, conduit à quatre stéréoisomères formant 2 coupies racémiques désignés par "Forme A", ou ""produit le plus polaire" et "Forme B", ou ""produit le moins polaire".

Sont particulierement intéreseants d'une façon générale, les produits de formule générale (I) qui se présentent sous la forme la plus polaire.

Parmi ces produits de formule générale (I) qui se présentent sous la forme la plus polaire, sont particulièrement intéressants ceux pour lesquels le symbole R représente un radical alcoyle contenant 1 à 3 atomes de carbone, Het représente un radical pyridy-le-3 ou quinolyle-3 et Y représente un radical méthylène.

Sont plus particulièrement intéressants ceux pour lesquels le symbole R représente un radical méthyle ou éthyle et Het représente un radical pyridyle-3 ou quinolyle-3 et Y représente un radical méthylène.

Sont d'un intérêt particulier, les produits suivants:

- le N-méthyl (pyridyl-3)-2 tétrahydrothiopyrannecarbothioamide-2-oxyde-1, Forme A (produit le plus polaire)
- le N-éthyl (pyridyl-5)-2 tétrahydrothiopyrannecarbothioamide-2-oxyde-1, Forme A (produit le plus polaire)
- le N-méthyl (quinolyi-3)-2 tétrahydrothiopyrannecarbothiosmide-2-oxyde-1, Forme A (produit le plus polaire)
- le N-méthyl (pyridyl-5)-2 tétrahydrothiophènecarbothioamide-2-oxyde-1, Forme A (produit le plus polaire).

Les exemples suivants, donnés à titre non limitatif, montrent comment l'invention peut être mise en pratique.

**EXEMPLE 1-**

A une solution de 10,2 g de (pyridyl-3)-2 tétrahydro-thiopyrannecarbodithioate-2-oxyde-1 de méthyle (une seule forme stéréoisomère) dans 150 $cm^5$ d'éthanol maintenue entre 25° et 30° C, on ajoute goutte à goutte et en 5 minutes 10,2 $cm^3$ d'une solution à 35% (poids/volume) de méthylamine dans l'éthanol. La solution est ensuite agitée pendant 1 heure et 35 minutes à une température voisine de 22° C puis on ajoute à nouveau 1,5 $cm^3$ de solution à 33% (poids/volume) de méthylamine dans l'ethanol. Après 1 heure d'agitation à la même température, le mélange réactionnel est concentré à sec sous pression réduite (25 mm de mercure; 3,4 kPa) à 40° C. Le produit obtenu (9,8 g) est dissous dans 15 $cm^3$ d'acétonitrile à une température voisine de 20° C et la solution est conservée pendant 1 heure à une température voisine de 5° C. Les cristaux apparus sont séparés par filtration, lavés 2 fois par 10 $cm^5$ au total d'acétonitrile et séchessous pression reduite (25 mm de mercure; 3,4 kPa) à 20° C. Le produit obtenu (3,4 g) auquel on a ajouté 0,8 g préparé dans les mêmes conditions, est dissous dans 80 $cm^3$ d'acétonitriie bouillant; la solution est additionnée de 0,1 g de noir décolorant, filtrée à chaud puis refroidie pendant 2 heures à une température voisine de 5° C, Les cristaux apparus sont séparés par filtration, lavés 2 fois par 10 $cm^3$ au total d'acétonitrile et

séchés sous pression réduits (1 mm de mercure; 0,13 kPa) à 60°C, On obtient ainsi 3,3 g de N-méthyl (pyridyl-3)-2 tétrahydrothiopyrannecarbothio-amids-2-oxyde-1, forme A,fondant a 228°C,[ R f = 0,22 (chromatographie sur couche mince de gel de silice; solvant:acétate d'éthyle-méthanol(80-20 en-volumes)].

Le (pyridyl-3)-2 tétrahydrothiopyrannecarbodithioate-2-oxyde-1 de méthyle (une seule forme stéréoisomère) peut être préparé de la manière suivante:

Une solution de 16,7 g de (chloro-4 butyl)-sulfinyl-méthyl-3 pyridine dans 90 $cm^3$ de tétrahydrofuranne anhydre est ajoutée goutte à goutte, en 20 minutes et en maintenant la température en-dessous de 12°C, à une solution de 16,2 g de tertbutylate de potassium dans un mélange de 90 $cm^3$ de tétrahydrofuranno et de 19 $cm^3$ d'hexaméthylphosphoramide anhydres, Après agitation pendant 30 minutes à une température voisine de 10°C, on ajoute goutte à goutte en 5 minutes et à la même température, 16,5 g de suifure de carbone en solution dans 15 $cm^3$ de tétrahydrofuranne anhydre. On agite pendant 10 minutes puis ajoute 30,8 g d'iodure de méthyle en solution dans 15 $cm^3$ de tétrahydrofuranne anhydre, Le mélange réactionnel est ensuite agité pendant 30 minutes en laissant remonter progressivement la température vers 20°C, Apres addition de 500 $cm^3$ d'eau distillée, on extrait 4 fois par 450 $cm^3$ au total d'acétate d'éthyle. Les extraits organiques sont réunis, lavés 4 fois par 600 $cm^3$ au total d'eau distillée, séchés sur du sulfate de sodium anhydre et concentrés à sec sous pression réduite (25 mm de mercure; 3,4 kPa) à 40°C, Le produit obtenu (12,6 g) est chromatographié sur 130 g de gel de silice neutre contenus dans une colonne de 3,7 cm de diamètre, On élue avec des mélanges de cyclohexane et d'acétate d'éthyle de teneur croissante en acétate d'éthyle pour éliminer les impuretés moins polaires que le produit attandu [Rf = 0,39; chromatographie sur couche mince de gel de silice; solvant:acétate d'éthyle-méthanol (80-20 en volumes)]. On élue ensuite avec de l'acetate d'éthyle pui en recueillant 14 fractions de 300 $cm^3$ qui sont réunies et concentrées à sec sous pression réduite (25 mm de mercure; 3,4 kPa) à 40°C.

On obtient ainsi 4,4 g de (pyridyl-3)-2 tétrahydrothiopy-rannecarbodithioate-2-oxyde-1 de méthyle (une seule forme stéréoisomère) fondant à 150°C,

La (chloro-4 butyl)-sulfinylméthyl-3 pyridine peut être préparée de la manière suivante:

A une solution de 103 g de sulfure de chloro-4 butyle et de pyridyl-3 méthyle dans 900 $cm^3$ de chlorure de méthylène on ajoute, goutte à goutte en 1 heure et 30 minutes à une température voisine de 20°C, une solution de 107 g d'acide m-chloroperbenzoïque à 85% dans 900 $cm^3$ de chlorure de méthylène. Après 17 heures d'agitation à la même température, on ajoute lentement 3 litres d'une solution aqueuse à 10% (poids) de bicarbonate de sodium, La solution organique est décantée puis lavée par 1 litre de solution à 10% de bicarbonate de sodium, Les phases aqueuses sont réunies et extraites par 500 $cm^3$ de chlorure de méthylène, Les extraits organiques sont réunis et lavés 2 fois par 2 litres au total d'eau distillée, séchés sur du sulfate de sodium anhydre et concentrés à sec sous pression réduite (25 mm de mercure; 3,4 kPa) à 35°C. Le produit obtenu (87 g) est chromatographié sur 400 g de gel de silice neutre contenus dans une colonne de 4,7 cm de diamètre, On élue avec 10 litres de chlorure de méthylène en recueillant successivement 3 fractions de 1 litre, 10 fractions de 100 $cm^3$ et 12 fractions de 500 $cm^3$. Les fractions 11 à 25 sont réunies et concentrées à sec sous pression réduite (25 mm de mercure; 3,4 kPa) à 35°C. On obtient ainsi 25,4 g de (chloro-4 butyl)-sulfinylméthyl-3 pyridine sous la forme d'une huile orange,

[Rf = 0,22; chromatographie sur couche mince de gel de silice; solvant: acétate d'éthyle - méthanol (80-20 en volumes)].

**EXEMPLE 2 -**

A une solution de 10,7 g de (pyridyl-3)-2 tétrahydrothio-pyrannecarbodithioate-2-oxyde-1 de méthyle dans 120 $cm^3$ d'éthanol maintenue entre 20 et 25°C, on ajoute goutte à goutte en 5 minutes 34 g d'éthylamine. La solution est ensuite agitée pendant 1 heure et 30 minutes à la même température puis concentrée à sec sous pression réduite (20 mm de mercure; 2,7 kPa) a 50°C,Le produit obtenu (12 g) est chromatographié sur 145 g de gel de silice neutre (0,040 - 0,063 mm) contenus dans une colonne-de 4 cm de diamètre sous une pression de 40 kPa, On élue la colonne par un mélange d'acétate d'éthyle et de méthanol (90 - 10 en volumes) en recueillant une fraction de 300 $cm^3$ et 32 fractions de 120 $cm^3$, Les fractions 16 à 33 sont réunies et concentrées à sec sous pression réduite (20 mm de mercure; 2,7 kPa) à 40°C. Le produit obtenu (4 g) est dissous dans un mélange bouillant de 9 $cm^3$ de méthyléthylcétone et de 2 $cm^3$ d'oxyde d'isopropyle; après refroidissement, la solution est conservée pendant 2 heures à une température voisine de 0°C, Les cristaux apparus sont séparés par filtration, lavés par 3 $cm^3$ de méthyléthylcétone et par 0,5 $cm^3$ d'oxyde d'isopropyle et séchés sous pression réduite (20 mm de mercure; 2,7 kPa) à une température voisine de 20°C. Le produit obtenu (2,6 g) additionné de 1,2 g préparé dans les mêmes conditions dans une opération antérieure est dissous dans 25 $cm^3$ de méthyléthylcétone bouillante; la solution, additionnée de 0,2 g de noir décolorant est filtrée à chaud, refroidie et conservée pendant 1 heure et 30 minutes à une tepérature voisine de 0°C. Les cristaux apparus sont séparés par filtration, lavés par 6 $cm^3$ de méthyléthylcétone et séchés sous pression réduite (1mm de mercure; 0,13 kPa) à 60°C. On obtient ainsi 3 g de N-éthyl (pyridyl-3)-2 tétrahydrothiopyrannecarbothioamide-2-oxyde-1-forme A, fondant d'abord vers 169°C, puis, après resolidification, à 183°C. [Rf = 0,30; chromatographie sur couche mince de gel de silice; solvant: acétate d'éthyle-méthanol (80-20 en volumes)].

**EXEMPLE 3 -**

En opérant comme à l'exemple 2 mais à partir de propylamine, on obtient la N-propyl (pyridyl-3)-2 tétrahydrothiopyrannecar-bothioamide-2-oxyde-1 fondant à 184°C. [Rf = 0,34; chromatographie sur couche mince de gel de silice; solvant: acétate d'éthyleméthanol (80-20 en volumes)].

**EXEMPLE 4 -**

A opérant comme à l'axemple 2 mais à partir d'isopropylamine, on obtient la N-isopropyl(pyridyl-3)-2 tétrahydrothiopyrannecar-bothioamide-2-oxyde-1 fondsnt à 209°C [Rf = 0,36; chromatographie sur couche mince de gel de silice; solvant: acétate d'éthyle - méthanol (80 - 20 en volumes)],

**EXEMPLE 5 -**

A une solution de 7,7 g de (quinolyl-3)-2 tétrahydrothio-pyrannecarbodithioate-2-oxyde-1 de méthyle dans 160 $cm^3$ d'éthanol maintenue à une température voisine de 20°C, on ajoute goutte à goutte en 25 minutes 55 $cm^3$ d'une solution à 33% (poids/volume) de méthylamine dans l'éthanol. La solution est ensuite agitée pendant 15 heures à la même température. Les cristaux apparus sont sépares par filtration, lavés par 10 $cm^3$ d'éthanol puis 3 fois par 45 $cm^3$ au total d'oxyde d'isopropyle et séchés sous pression réduite (0,2 mm de mercure; 0,027 kPa) à 50°C. Le produit obtenu (3,8 g) additionné de 0,95 g préparé dans les mêmes conditions dans une autre opération antérieure,est dissous dans 600 $cm^3$ de méthanol bouillant; la solution, additionnée de 4 g de noir décolorant est filtrée à chaud, refroidie puis conservée à une température voisine de 5°C pendant 15 heures. Le solide apparu est séparé par filtration, lavé 4 fois par 80 $cm^3$ au total de méthanol et séché sous pression réduite (0,2 mm de mercure; 0,027 kPa) à une température voisine de 60°C. On obtient ainsi 4,3 g de N-méthyl (quinolyl-3)-2 tétrahydrothiopyrannecarbothioamide-2-oxyde-1 fondant à 280°C. [Rf = 0,38; chromatographie sur couche mince de gel de silice; solvant: acétate d'éthyle-méthanol (80-20 en volumes)].

Le (quinolyl-3)-2 tétrahydrothiopyrarnecarboditdioate-2-oxyde-1 de méthyle peut être préparé de la façon suivante: Une suspension de 34,5 g de (quinolyl-3)-2 tétrahydro-thiopyranne-oxyde-1 dans 280 $cm^3$ de tétrahydrofuranne anhydre est ajoutée goutte à goutte, en 45 minutes à une température voisine de 20°C, à une solution de 34,9 g de tert-butylate de potassium dans 800 $cm^3$ de tétrahydrofuranne anhydre. Le mélange est ensuite agité pendant 1 heure à la même température: on ajoute alors goutte à goutte en 15 minutes a une température de −4°C une solution de 27 g de sulfure de carbone dans 60 $cm^3$ de tétrahydrofurane anhydre. Après 30 minutes d'agitation on ajoute goutte à goutte en 15 minutes à la même température une solution de 51 g d'iodure de méthyle dans 60 $cm^3$ de tétrahydrofuranne anhydre. Le mélange est ensuite agité pendant 30 minutes en laissant remonter progressivement la température à 12°C. Le solide apparu est sépare par filtration, lavé par 200 $cm^3$ de chlorure de méthylène et éliminé. Le filtrat et les liqueurs de lavage sont réunis et concentrés à sec sous pression réduite (25 mm de mercure; 3,4 kPa) à 45°C. Le produit obtenu (51 g) est dissous dans 250 $cm^3$ de chlorure de méthylène; la solution est lavée par 150 $cm^3$ d'eau distillée, séchée sur du sulfate de magnésium anhydre et versée sur 770 g de gel de silice neutre (0,063 - 0,200 mm) contenus dans une colonne de 7 cm de diamètre, La colonne est éluée avec 13 litres d'un mélange de chlorure de méthylène et d'acétate d'éthyle (50 - 50 en volumes) en recueillant des fractions de 1000 $cm^3$. Les fractions 9 à 13 sont réunies et concentrées à sec sous pression réduite (20 mm de mercure; 2,7 kPa) à 40°C. On obtient ainsi 7,85 g de (quinolyl-3)-2 tétrahydrothio-pyrannecarbodithioate-2-oxyde-1 de méthyle fondant à 210°C. [Rf = 0,39; chromatographie sur couche mince de gel de silice; solvant: acétate d'éthyle ].

Le (quinolyl-3)-2 tétrahydrothiopyranne-oxyde-1 peut être obtenu de la façon suivante:

Une solution de 58,4 g de (chloro-4 butyl) sulfinyl-méthyl-3 quinoléine dans 450 $cm^3$ de tétrahydrofuranne anhydre est ajoutée goutte à goutte en 1 heure et 15 minutes à une température voisine de 0°C à une solution de 44,8 g de tert-butylate de potassium dans 200 $cm^3$ de tétrahydrofuranne anhydre. Le mélange est ensuite agité pendant 16 heures en laissant remonter la température à 18°C; on ajoute alors goutte à goutte en 15 minutes à une température comprise entre 2 et 10°C 15 $cm^3$ d'acide acétique. Le solide apparu est séparé par filtration, lavé 2 fois par 360 $cm^3$ au total de chlorure de méthylène et éliminé. Le filtrat et les liqueurs de lavage sont réunis et concentrés à sec sous pression réduite (20 mm de mercure; 2,7 kPa) à 45°C, Le produit obtenu (51 g) est chromatographié sur 185 g de gel de silice neutre (0,063 - 0,200 mm) contenus dans une colonne de 6,8 cm de diamètre. La colonne est éluée par 200 $cm^3$ de chlorure de méthylène, puis 2200 $cm^3$ d'acétate d'éthyle et enfin 1600 $cm^3$ d'un mélange d'acétate d'éthyle et de methanol (90 - 10 en volumes) en recueillant 40 fractions de 100 $cm^3$. Les fractions 25 à 40 sont réunies et concentrées à sec sous pression réduite (20 mm de mercure; 2,7 kPa) à 45°C. On obtient ainsi 35,8 g du mélange des 2 formes de (quinolyl-3)-2 tétrahydrothiopyranne-oxyde-1 fondant à 152°C - 154°C.

[Rf = 0,09 et 0,17; chromatographie sur couche mince de gel de silice; solvant: acétate d'éthyle].

La (chloro-4 butyl) sulfinylméthyl-3 quinoléine peut être préparée de la façon suivante:

A une solution de 54,8 g de sulfure de chloro-4 butyle et de quinolyl-3 méthyle dans 1160 $cm^3$ de chlorure de

méthylène, on ajoute, goutte à goutte en 1 heure et 15 minutes, à une température voisine de 20°C, une solution de 44,4 g d'acide m-chloroperbenzoïque à 82,5% dans 450 $cm^3$ de chlorure de méthylène. Apres 18 heures d'agitation à la même température, on ajoute lentement 560 $cm^3$ d'une solution aqueuse à 8% (poids/volume) de bicarbonate de sodium. La solution organique est décantée, lavée par 200 $cm^3$ de solution aqueuse à 8% de bicarbonate de rodium puis 2 fois par 300 $cm^3$ au total d'eau distillée, séchée sur du sulfate de magnésium anhydre et concentrée à sec sous pression réduite (20 mm de mercure; 2,7 kPa) à 45°C. On obtient ainsi 58,1 g de (chloro-4 butyl) sulfinylméthyl-3 quinoléine fondant à 103°C.

Le sulfure de chloro-4 butyle et de quinolyl-3 méthyle peut être préparé de la façon suivante:

A une solution maintenue a une température voisine da 0°C, de 87 g de dichlorhydrate de (quinolyl-3 méthyl)-2 isothiourée dans 200 $cm^3$ d'eau distillée on ajoute, en 12 minutes, 93 $cm^3$ d'une solution aqueuae de aoude 10 N, Après chauffage pendant 20 minutes à une température voisine de 70°C puis refroidissement à 12°C, on ajoute 3 g de chlorure de triéthylbenzylammonium, 125 $cm^3$ de chlorure de méthylène puis, goutte à goutte à une température voisine de 4°C, 51,5 g de bromo-1 chloro-4 butane et poursuit l'agitation pendant 16 heures à une température voisine de 20°C. La phase organique est séparée par décantation, et la phase aqueuse est extraite 2 fois par 100 $cm^3$ au total de chlorure de méthylène. Les extraits organiques sont réunis, lavés 2 fois par 100 $cm^3$ au total d'eau distillée, séches sur du sulfate de magnésium anhydre et versés sur 230 g de gel de silice neutre (0,063 - 0,200 mm) contenus dans une colonne de 4,2 cm de diamètre; la colonne est éluée par 1380 $cm^3$ de chlorure de méthylène en recueillant 1 fraction de 220 $cm^3$ qui est éliminée et une de 1160 $cm^3$ qui contient 54,8 g de sulfure de chloro-4 butyle et de quinolyl-3 méthyle (poids déterminé par dosage perchlorique de la solution) et qui sera utilisée sans isolement du produit pour la suite de la synthèse.

[Rf = 0,65; chromatographie sur couche mince de gel de silice; solvant: acétate d'éthyle].

Le dichlorhydrate de (quinolyl-3 méthyl)-2 isothiourée peut être préparé comme décrit dans la demande de brevet européen publiée sous le numéro 0046.417.

**EXEMPLE 6 -**

A une solution de 0,35 g de (pyridyl-3)-2 tétrahydro-thiopyrannecarbodithioate-2-oxyde-1 de méthyle [forme la moins polaire; Rf = 0,13; chromatographie sur couche mince de gel de silice; solvant: acétate d'éthyle], dans 2 $cm^3$ d'éthanol on ajoute goutte a goutte en 5 minutes à une température voisine de 20°C, 2 $cm^3$ d'une solution à 33% (poids/ volume) de méthylamine dans l'éthanol. Après 2 heures d'agitation à la même température, le mélange réactionnel est concentré à sec sous pression réduite (20 mm de mercure; 2,7 kPa) à 30°C. Le produit obtenu (0,28 g) est chromatographié sur 35 g de gel de silice neutre (0,040 - 0,063 mm) contenus dans une colonne de 2 cm de diamètre sous une pression de 40 kPa. On élue successivement par 100 $cm^3$ de chlorure de méthylène, 100 $cm^3$ d'acétate d'éthyle et 100 $cm^3$ d'un mélange d'acétate d'éthyle et de méthanol (90 - 10 en volumes) en recueillant 11 fractions de 20 $cm^3$. Les fractions 6 à 11 sont réunies et concentrées à sec sous pression réduite (20 mm de mercure; 2,7 kPa) à 40°C. On obtient ainsi 0,22 g de N-méthyl (pyridyl-3)-2 tétrahydrothiopyrannecarbothioamide-2-oxyde-1 brut, forme B, la moins polaire.

[Rf = 0,48; chromatographie sur couche mince de gel de silice; solvant: acétate d'éthyle-méthanol (80 - 20 en volumes)].

Le (pyridyl-3)-2 tétrahydrothiopyrannecarbodithioate-2-oxyde-1 de méthyle peut être préparé de la façon suivante.

A une solution de 1,6 g de (pyridyl-3)-2 tétrahydrothio-pyrannecarbodithioate-2 de methyle dans 15 $cm^3$ de chlorure de méthylène on ajoute goutte à goutte en 30 minutes à une température voisine de 20°C, une solution de 1,25 g d'acide m-chloroperbenzoique à 82,5% dans 10 $cm^3$ de chlorure de methylène. Après 20 heures d'agitation à la même température, le mélange réactionnel est versé sur 40 g de gel de silice neutre contenus dans une colonne de 2,6 cm de diamètre. La colonne est ensuite éluée successivement par 300 $cm^3$ de chlorure de méthylène, 400 $cm^3$ d'un mélange de chlorure de méthylène et d'acétate d'éthyle (50 - 50 en volumes) et par 1 litre d'acétate d'éthyle en recueillant successivement une fraction de 300 $cm^3$, une fraction de 400 $cm^3$ et 39 fractions de 25 $cm^3$.

Les fractions 8 à 28 sont réunies et concentrées à sec sous pression réduite (15 mm de mercure; 2 kPa)à 40°C. On obtient ainsi 0,5 g de la forme la moins polaire du (pyridyl-3)-2 tétrahydrothiopyrannecarbodithioate-2-oxyde-1 de méthyle [Rf = 0,13; chromatographie sur couche mince de gel de silice; solvant: acétate d'éthyle].

Les fractions 30 à 41 sont réunies et concentrées a sec sous pression réduite (15 mm de mercure; 2 kPa) à 40°C. On obtient ainsi 1 g de la forme la plus polaire du (pyridyl-3)-2 tétrahydrothiopyrannecarbodithioate-2-oxyde-1 de méthyle [Rf = 0,07; chromatographie sur couche mince de gel de silice; solvant: acétate d'éthyle] identique au dithioester dont la preparation est donnée à l'exemple 1.

Le (pyridyl-3)-2 tétrahydrothiopyrannecarbodithioate-2 de méthyle peut être préparé de la manière suivante:

A 47 $cm^3$ d'une solution 1,6 M de n-butyllithium dsns l'hexane, maintenue sous atmosphère d'azote et refroidie à -61°C, on ajoute goutte à goutte en 7 minutes une solution de 7,6 g de diisopropylamine dans 30 cm d'un mélange d'hexaméthylphosphoramide et de tétrahydrofuranne anhydres (47-53 en volumes). On ajoute ensuite, en 10 minutes une solution de 6,45 g de sulfure de chloro-4 butyle et de pyridyl-3 méthyle dans 30 $cm^3$ du mélange d'hexaméthylphosphoramide et de tétrahydrofuranne anhydres (47-53 en volumes). Après 1 heure d'agitation à une température voisine de -60°C, on ajoute en 10 minutes 9,1 g de sulfure de carbone en solution

dans 30 $cm^3$ du mélange d'hexaméthylphosphoramide et de tétrahydrofuranne anhydres (47-53 en volumes). Après 10 minutes d'agitation à la même température, on ajoute en 10 minutes, 17 g d'iodure de méthyle en solution dans 30 $cm^3$ du mélange d'hexaméthylphosphoramide et de tétrahydrofuranne anhydres (47-53 en volumes). Le mélange réactionnel est ensuite agité pendant 30 minutes à une température voisine de −60°C puis pendant 60 minutes en laissant remonter progressivement la température jusqu'à 10°C. Après addition de 100 $cm^3$ d'eau distillée, le mélange réactionnel est extrait 5 fois par 400 $cm^3$ au total d'acétate d'éthyle. Les extraits organiques sont réunis et lavés 3 fois par 300 $cm^3$ au total d'eau distillée. Après séchage sur du sulfate de sodium anhydre, filtration et concentration à sec, on obtient 15,9 g d'une huile brune que l'on chromatographie sur 160 g de gel de silice neutre contenus dans une colonne de 3,7 cm de diamètre. On élue avec 500 $cm^3$ de cyclohexane, 400 $cm^3$ d'un mélange de cyclohexane et d'acétate d'éthyle (98-2 en volumes), 400 $cm^3$ d'un mélange de cyclohexane et d'acétate d'éthyle (95-5 en volumes), 400 $cm^3$ d'un mélange de cyclohexane etd'acétate d'éthyle (92-8 en volumes), 400 $cm^3$ d'un mélange de cyclohexane et d'acétate d'éthyle (90 - 10 en volumes),1000 $cm^3$ d'un mélange de cyclohexanne et d'acétate d'éthyle (88-12 en volumes) en recueillant 1 fraction de 500 $cm^3$, 8 fractions de 200 $cm^3$ et 10 fractions de 100 $cm^3$. Les fractions 12 à 19 sont réunies et concentrées à sec. On obtient ainsi 2,1 g de (pyridyl-3)-2 tétrahydrothiopyrannecarbodithioate-2 de méthyle fondant à 90°C.

**EXEMPLE 7 -**

A une solution de 12,5 g de (pyridyl-3)-2 tétrahydro-thiopyrannecarbodithioate-2 de méthyle dans 120 $cm^3$ de chlorure de méthylène on ajoute, goutte à goutte en 45 minutes à une température voisine de 20°C, une solution de 9,8 g d'acide m-chloroperbenzoïque à 82,5% dans 130 $cm^3$ de chlorure de méthylène. Après 21 heures d'agitation à la même température, le mélange réactionnel est versé sur 375 g de gel de silice neutre (0,063 - 0,200 mm) contenus dans une colonne de 5 cm de diamètre; la colonne est ensuite éluée successivement par 1 litre de chlorure de méthylène, par 600 $cm^3$ d'acétate d'éthyle et par 800 $cm^3$ d'un mélange d'acetate d'éthyle et de méthanol (80 - 20 en volumes) en recueillant une fraction de 1 litre et 7 fractions de 200 $cm^3$. Les fractions 5 à 8 sont réunies et concentrées à sec sous pression reduite (15 mm de mercure; 2 kPa) à 40°C. On obtient ainsi 12,5 g du mélange des 2 formes du (pyridyl-3)-2 tétrahydrothiopyrannecarbodithio-ste-2-oxyde-1 de méthyle. A une solution de 6 g de ce mélange dans 50 $cm^3$ d'éthanol, on ajoute goutte à goutte en 35 minutes à une température voisine de 20°C, 35 $cm^3$ d'une solution à 33% (poids/ volume) de méthylamine dans l'éthanol. Après 4 heures d'agitation à la même température, le mélange réactionnel est concentré à sec sous pression réduite (20 mm de mercure; 2,7 kPa) à 45°C. On obtient ainsi 6,2 g de produit brut présentant en chromatographie sur conche mince de gel de silice, deux tâches correspondant à des Rf de 0,48 et 0,22 [solvant d'élution: acétate d'éthyleméthanol (80-20 en volumes)]. La séparation des deux produits correspondants s'effectue de la manière suivante:

Le produit brut obtenu est dissous dans 55 $cm^3$ de chlorure de méthylène et la solution est versée sur 100 g de gel de silice neutre (0,040 - 0,063 mm) contenus dans une colonne de 2,6 cm de diamètre; la colonne est éluée sous une pression de 40 kPa par 480 $cm^3$ d'acétate d'éthyle en recueillant 6 fractions de 80 $cm^3$. Les fractions 4 à 6 sont réunies et concentrées à sec sous pression réduite (15 mm de mercure; 2 kPa) à 40°C. Le produit obtenu (1,1 g), auquel on a ajouté 1,7 g du même produit préparé dans les mêmes conditions dans une opération antérieure, est dissous dans 25 $cm^3$ de chlorure de méthylène; la solution est additionnée de 0,5 g de noir décolorant, filtrée et, après 3 lavages par 15 $cm^3$ au total de chlorure de méthylène, additionnée de 125 $cm^3$ de cyclohexane puis conservée pendant 15 heures à une température voisine de 5°C. Les cristaux apparus sont séparés par filtration, lavés 5 fois par 50 $cm^3$ au total de cyclohexane et séchés sous pression réduite (0,1 mm de mercure; 0,013 kPa) à 50°C. On obtient ainsi 1,9 g de la forme B du N-méthyl (pyridyl-3)-2 tétrahydrothiopyrannecarbothioamide-2-oxyde-1, forme la moins polaire [Rf = 0,48; chromatographie sur couche mince de gel de silice; solvant: acétate d'éthyle-méthanol (80-20 en volumes)] fondant à 154°C.

En poursuivant la chromatographie, on peut obtenir la forme A, forme la plus polaire [Rf = 0,22; chromatographie sur couche mince de gel de silice; solvant: acétate d'éthyle-méthanol (80-20 en volumes)]fondant à 228°C.

**EXEMPLE 8 -**

A une solution de 2,7 g de (pyridyl-3)-2 tétrahydrothio-phènecarbodithioate-2-oxyde-1 de méthyle (mélange des deux formes stéréoisomères), dans 25 $cm^3$ d'éthanol, on ajoute 2,7 g de chlorure mercurique, 5 $cm^3$ d'eau distillée puis, goutte à goutte, en 10 minutes et a une température voisine de 15°C, 5 $cm^3$ d'une solution a 33% (poids/volume) de méthylamine dans l'éthanol. Le mélange est agité pendant 30 minutes a la même température; on ajoute à nouveau 3 $cm^3$ de la solution de méthylamine dans l'éthanol. Après 30 minutes d'agitation, l'insoluble apparu est éliminé par filtration et le filtrat est concentré à sec sous pression réduite (20 mm de mercure; 2,7 kPa) à 40°C. On obtient ainsi 5 g de produit brut présentant en chromatographie sur couche mince de gel de silice deux tâches correspondant à des Rf de 0,20 et 0,13 [solvant d'élution: acétate d'éthyle-méthanol(80-20 en volumes)]. La séparation des produits correspondants s'effectue de la manière suivante: Le

produit brut obtenu est additionné de 10 $cm^3$ de méthanol; la solution est filtrée,puis versée sur 75 g-de gel de silice neutre (0,040 - 0,063 mm) contenus dans une colonne de 2,5 cm de diamètre, et éluée sous une pression de 50 kPa. On élue successivement par 2040 $cm^3$ d'un mélange d'acétate d'éthyle et de méthanol (95-5 en volumes) puis par 960 $cm^3$ d'un mélange d'acétate d'éthyle et de méthanol (90-10 en volumes) en recueillant des fractions de 60 $cm^3$.

a) Les fractions 19 à 31 sont réunies et concentrées à sec sous pression réduite (20 mm de mercure; 2,7 kPa) à 40°C. On obtient ainsi 0,7 g de produit que l'on purifie comme suit:

Le produit est mis en suspension dans 15 $cm^3$ d'acétonitrile bouillant et la solution est filtrée à chaud; le filtrat est concentré à sec sous pression réduite (20 mm de mercure;2,7 kPa) à 40°C. Le produit obtenu (0,4 g) est dissous dans 4 $cm^3$ d'éthanol bouillant. Après refroidissement et conservation pendant 30 minutes à 0°C, les cristaux apparus sont séparés par filtration, lavés 2 fois par 1 $cm^3$ au total d'éthanol et séchés sous pression réduite (20 mm de mercure; 2,7 kPa) à une température voisine de 20°C. On obtient ainsi 0,15 g de N-méthyl (pyridyl-3)-2 tétrahydrothiophène carbothioamide-2-oxyde-1, forme B fondant à 158°C [Rf = 0,20; chromatographie sur couche mince de gel de silice; solvant: acétate d'éthyle - méthanol (80-20 en volumes)].

b) Les fractions 38 à 50 sont réunies et concentrées a sec sous pression reduite (20 mm de mercure;2,7 kPa);on obtient ainsi 0,65 g de produit que l'on purifie comme suit:

Le produit est mis en suspension dans 12 $cm^3$ d'acétonitrile bouillant et la solution est filtrée à chaud; le filtrat est concentré à sec sous pression réduite (20 mm de mercure; 2,7 kPa) à 40°C. Le produit obtenu (0,4 g) est dissous dans 2,5 $cm^3$ d'acétonitrile bouillant. Après refroidissement et conservation pendant 30 minutes à 0°C, les cristaux apparus sont séparés par filtration, lavés 2 fois par 1 $cm^3$ au total d'acétonitrile et séchés sous pression réduite (20 mm de mercure; 2,7 kPa) à une température voisine de 0°C. On obtient ainsi 0,14 g de N-méthyl (pyridyl-3)-2 tétrahydrothiophènecarbothioamide-2-oxyde-1, forme A, fondant à 204°C [Rf = 0,13; chromatographie sur couche mince de gel de silice; solvant: acétate d'éthyle - méthanol (80-20 en volumes)].

Le (pyridyl-3)-2 tétrahydrothiophènecarbodithioate-2-oxyde-1 de méthyle (mélange des deux formes stéréoisomères) peut être préparé de la manière suivante:

A une solution de 11,2 g de (pyridyl-3)-2 tétrahydro-thiophènecarbodithioate-2 de méthyle dans 150 $cm^3$ de chlorure de méthylène,on ajoute goutte à goutte en 1 heure à une température voisine de 20°C une solution de 8,9 g d'acide m-chloroperbenzoïque à 85% dans 150 $cm^3$ de chlorure de méthylène. Après 17 heures d'agitation à la même température, le mélange réactionnel est concentré à sec sous pression réduite (20 mm de mercure; 2,7 kPa) à 50°C puis chromatographié sur 550 g de gel de silice neutre (0,040-0,063 mm) contenus dans une colonne de 6 cm de diamètre sous pression d'azote (50 kPa). On élue successivement par 1,2 litre d'acétate d'éthyle par 1,2 litre d'un mélange d'acétate d'éthyle et de méthanol (95-5 en volumes) et par 3,9 litres d'un mélange d'acétate d'éthyle et de méthanol (90-10 en volumes) en recueillant une fraction de 2,5 litres et 35 fractions de 100 $cm^3$. Les fractions 13 à 35 sont réunies et concentrées à sec sous pression réduite (20 mm de mercure; 2,7 kPa) à 50°C.

On obtient ainsi 10,2 g de (pyridyl-3)-2 tétrahydrothiophènecarbo-dithioate-2-oxyde-1 de méthyle, sous forme d'une huile orangée correspondant a un mélange dans la proportion de 50% des deux formes stéréoisomères [proportion déterminée par RMN sur le proton $H_2$ dédoublé de la pyridine: Ç = 8,85 et 8,77 ppm; J = 2,5 Hz (solvant $CDCl_3$)] et ayant un Rf unique égal à 0,37 [chromatographie sur couche mince de gel de silice; solvant acétate d'éthyle-méthanol (80-20 en volumes)].

**EXEMPLE 9 -**

A une solution de 5,8 g de (pyridyl-3)-2 tétrahydrothio-phènecarbodithioate-2-oxyde-1 de méthyle (une seule forme stéréoisomère) dans 35 $cm^3$ d'éthanol maintenue à une température voisine de 25°C, on ajouta goutte à goutte en 16 minutes 15 $cm^3$ d'une solution à 33% (poids/volume) de méthylamine dans l'éthanol. Après 1 heure d'agitation à la même température, on ajoute goutte à goutte en 10 minutes 15 $cm^3$ de la solution à 33% (poids/volume) de méthylamine dans l'éthanol. Après 50 minutes d'agitation, le mélange réactionnel est concentré à sec sous pression réduite (20 mm de mercure; 2,7 kPa) à 40°C. Le produit obtenu (7,4 g) est chromatogaphié sur 190 g de gel de silice neutre (0,040 - 0,063 mm) contenus dans une colonne de 30 cm de diamètre. On élue sous pression (50 kPa) successivement par 500 $cm^3$ d'acétate d'éthyle, 1360 $cm^3$ d'un mélange d'acétate d'éthyle et de méthanol (97-3 en volumes), 760 $cm^3$ d,un mélange d'acétate d'éthyle et de méthanol (95-5 en volumes) et 1240 $cm^3$ d'un mélange d'acétate d'éthyle et de méthanol (90-10 en volumes) en recueillant 5 fractions de 100 $cm^3$ et 84 fractions de 40 $cm^3$. Les fractions 53 à 89 sont réunies et concentrées à sec sous pression réduite (20 mm de mercure; 2,7 kPa) à 40°C. Le produit obtenu (1,2 g) est dissous dans 3,5 $cm^3$ d'éthanol bouillant et après refroidissement et conservation pendant 30 minutes à 0°C, les cristaux apparus sont séparés par filtration, lavés 2 fois par 1 $cm^3$ au total d'éthanol et séchés sous pression réduite (1 mm de mercure; 0,13 kPa) à 60°C. On obtient ainsi 0,28 g de Nméthyl (pyridyl-3)-2 tétrahydrothiophènecarbothioamide-2-oxyde-1, (forme B, la moins polaire)fondant à 156°C [Rf, 0,20; chromatographie sur couche mince de gel de silice; solvant: acétate d'éthyle - méthanol (80-20 en volumes)].

Le (pyridyl-3)-2 tétrahydrothiophènecarbodithioate-2-oxyde-1 de méthyle peut être préparé de la manière suivante:

A une solution de 14,8 g de tert-butylate de potassium dans 150 $cm^3$ de tétrahydrofuranne anhydre, on ajoute

goutte à goutte en 20 minutes à une température voisine de 20°C, une solution de 18,1 g de (pyridyl-3)-2 tétrahydrothiophène-oxyde-1 dans 150 $cm^3$ de tétrahydrofuranne anhydre. Après 40 minutes d'agitation, on ajoute goutte à goutte en 15 minutes à -20°C une solution de 14 $cm^3$ de sulfure de carbone dans 100 $cm^3$ de tétrahydrofuranne anhydre. Après 25 minutes d'agitation, on ajoute goutte à goutte en 10 minutes à la même température une solution de 14,5 $cm^3$ d'iodure de méthyle dans 100 $cm^3$ de tétrahydrofursnne anhydre. Le mélange réactionnel est ensuite agité pendant 2 heures et 40 minutes en laissant remonter progressivement la température à 15°C. L'insoluble apparu est séparé par filtration et lavé 2 fois par 120 $cm^3$ au total de tétrahydrofuranne anhydre. Le filtrat et les liqueurs de lavage sont réunis et concentrés à sec sous pression réduite (20 mm de mercure; 2,7 kPa) à 40°C. Le produit obtenu (25,8 g) est chromatographié sur 500 g de gel de silice neutre (0,040 -0,063 mm) contenus dans une colonne de 6 cm de diamètre. On élue sous pression (50 kPa) par 6,6 litres d'un mélange d'acétate d'éthyle et de méthanol (93-7 en volumes) en recueillant 14 fractions de 250 $cm^3$ et 31 fractions de 100 $cm^3$. Les fractions 18 à 36 sont réunies et concentrées à sec sous pression réduite (20 mm de mercure; 2,7 kPa) à 40°C. On obtient ainsi 5 g de (pyridyl-3)-2 tétrahydrothiophènecarbodithioate-2-oxyde-1 de méthyle [Rf = 0,30; chromatographie sur couche mince de gel de silice; solvant: acétate d'éthyle - méthanol (80-20 en volumes)].

Le (pyridyl-3)-2 tétrahydrothiophène-oxyde-1 peut être préparé de la manière suivante:

A une solution de 62 g de (pyridyl-3)-2 tétrahydrothiophène dans 650 $cm^3$ de chlorure de méthylène, on ajoute goutte à goutte en 2 heures et 10 minutes à une température voisine de 20°C, une solution de 76,3 g d'acide m-chloroperbenzoïque à 85% dans 800 $cm^3$ de chlorure de méthylène. Après 16 heures d'agitation à la même températurs, le mélange réactionnel est concentré au 1/4 environ de son volume initial sous pression réduite et le concentrat est versé sur 800 g de gel de silice neutre (0,063 - 0,200 mm) contenus dans une colonne de 8 cm de diamètre. On élue successivement par 9 litres de chlorure de méthylène et par 7 litres d'un mélange de chlorure de méthylène et de méthanol (95-5 en volumes) en recueillant 16 fractions de 1 litre. Les fractions 15 et 16 sont réunies et concentrées à sec sous pression réduite (20 mm de mercure; 2,7 kPa) à 40°C. Le produit obtenu (54,3 g) est dissous dans 100 $cm^3$ de chlorure de méthylène et la solution est lavée 2 fois par 100 $cm^3$ au total d'une solution aqueuse à 10% de bicarbonate de sodium. Après décantation, les eaux de lavage sont extraites 3 fois par 300 $cm^3$ au total de chlorure de méthylène. Les extraits organiques sont réunis, séchés sur du sulfate de sodium anhydre et concentrés à sec sous pression réduite (20 mm de mercure; 2,7 kPa) à 40°C. On obtient ainsi 32,8 g de (pyridyl-3)-2 tétrahydrothiophè-ne-oxyde-1, mélange des 2 formes stéréoisomères [Rf = 0,36 et 0,40; chromatographie sur couche mince de gel de silice; solvant: acétate d'éthyle - méthanol - eau - acide acétique (70-20-5-5 en volumes)].

Le (pyridyl-3)-2 tétrahydrothiophène peut être préparé selon la methode decrite dans la demande de brevet européen publiée sous le n° 0046.417.

La présente invention concerne également les médicaments constitués par-au moins un produit de formule générale (I), à l'état pur ou sous forme d'une composition dans laquelle il est associé à tout autre produit pharmaceutiquement compatible, pouvant être inerte ou physiologiquement actif. Les médicamente selon l'invention peuvent être utilisés par voie orale, parentérale ou rectale.

Comme compositions solides pour administration orale peuvent être utilisés des comprimés, pilules, poudres (notamment dans des capsules de gelatine ou des cachets) ou granulés. Dans ces compositions, le produit actif selon l'invention est mélange à un ou plusieurs diluants inertes, tels que amidon, cellulose, saccharose, lactose ou silice. Ces compositions peuvent également comprendre des substances autres que les diluants, par exemple un ou plusieurs lubrifiants tel que le stéarate de magnesium ou le talc, un colorant, un enrobage (dragées) ou un vernis.

Comme compositions liquides pour administration orale, on peut utiliser des solutions, des suspensions, des émulsions, des sirops et des élixiis pharmaceutiquement acceptables contenant des diluants inertes tels que l'eau. l'éthanol, le glycérol, les huiles végétales ou l'huile de paraffine. Ces compositions peuvent également comprendre des substances autres que les diluants, par exemple des produits mouillants, édulcorants, épaississants, aromatisants ou stabilisants.

Les compositions stériles pour administration parentérale peuvent être de préference des solutiohs aqueuses ou non aqueuses, des suspensions ou des émulsions. Comme solvant ou vehicule, on peut employer l'eau, le propylèneglycol, un polyéthylèneglycol, des huiles vegetales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forms de compositions solides stérilas qui peuvent être dissoutes au moment de l'emploi dans un milieu stérile injectable.

Les compositions pour admimistration rectale sont les suppositoires ou lss capsules rectales, qui contiennent outre le produit actif des excipients tels que le beurre de cacao, des glycérides semi-synthétiques ou des polyéthylèneglycols.

En thérapeutique humaine, les produits de l'invention sont particulièrement utiles dans le traitement de l'hypertension.

Les doses dépendent de l'effet recherché et de la durée du traitement; elles sont généralement comprises entre 5 et 1000 mg par jour par voie orale pour un adulte en une ou plusieurs prises.

D'une façon générale, le médecin déterminera la posologie qu'il estime la plus apprcpriée en fonction de l'âge

du poids et de tous les autres facteurs propres au sujet à traiter.

Les exemples suivants donnés à titre non limitatif, illustrent une composition selon l'invention.

**EXEMPLE A -**

On prépare selon la technique habituelle des comprimés dosés à 25 mg de produit actif ayant la composition suivante:

-N-méthyl (pyridyl-3)-2 tétrahydrothiopyrannecarbo-thiosmide-2-oxyde-1, Forme A (produit le plus polaire)..........25 mg
-amidon..........60 mg
-silice co1loïdale..........50 mg
-stéréarate de magnésium.......... 2 mg

**EXEMPLE B -**

On prépare selon la technique habituelle des commrimés dosés à 25 mg de produit actif ayant la composition suivante:

-N-méthyl (quinolyl-3)-2 tétrahydrothiopyrannecarbothioamide
2-oxyde-1................. 25 mg
- amidon................. 60 mg
- silice colloïdale................. 50 mg
- stearate de magnésium................. 2 mg

**Revendications** pour les Etats contractants BE CH DE FR GB IT LI LU NL SE

1 - Dérivé de la thioformamide caractérisé en ce qu'il répond à la formule générale:

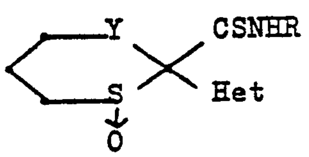

dans laquelle R représente un atome d'hydrogène ou un radical alcoyle contenAnt 1 à 4 atomes de carbone en chaîne droite ou ramifiée, Het représente un radical hétérocyolîque à oaractèrea aromatique contenant un atome d'azote choisi parmi pyridyle-3 ou quinolyle-3, et Y représente une lisison de valence ou un radical méthylène.

2 - Procédé de préparation d'un produit selon la revendication 1 caractérisé en ce que l'on fait réagir une amine de formule générale:

$R - NH_2$

dans laquelle R est défini comme dans la revendication 1 sur un dithioester de formule générale:

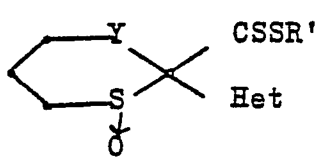

dans laquelle les symboles Y et Het sont définis comme dans la revendication 1 et R' représente un radical alcoyle contenant 1 à 4 atomes de carbone en chaine droite ou ramifiée ou un radical benzyle ou carboxyméthyle, puis on isole le produit obtenu et sépare, le cas échéant, les formes stéréoisomères.

3 - Procédé selon la revendication 2, caractérisé en ce que l'on fait réagir l'amine de formule générale:

$R - NR_2$

sur un dithioester de formule générale:

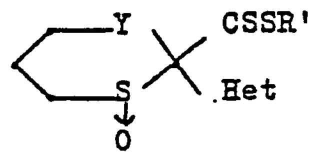

dont on a préslablement séparé les formes diastéréoisomères.

4 - Médicament caractérisé en ce qu'il contient un produit selon la revendication 1 éventuellement en asscoiation avec un ou plusieurs diluants ou adjuvants compatibles et pharmaceutiquement acceptables.

**REVENDICATONS** pour l'Etat contractant AT

1 - Procédé de préparation d'un nouveau dérivé de la thioformamide de formule générale:

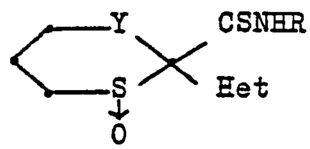

dans laquelle R représente un atome d'hydrogène ou un radical alcoyle contenant 1 à 4 atomes de carbone en chaîne droite ou ramifiée, Ret représente un radical hétérocyclique à caractère aromatique contenant un ou deux atomes d'azote choisi parmi pyri-dyle-3 ou quinolyle-3, et Y représente une liaison de valence ou un radical méthylène caractérisé en ce que l'on fait réagir une amine de formule générale:

$R - NH_2$

dans laquelle R est défini comme ci-dessus sur un dithioester de formule générale:

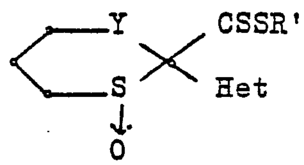

dans laquelle les symboles Y et Het sont définis comme précédemment et R' représente un radical alcoyle contenant 1 à 4 atomes de carbone en chaîne droite ou ramifiée ou un radical benzyle ou carboxyméthyle, puis on isole le produit obtenu et sépare, le cas échéant, les formes stéréoisomères.

2 - Procédé selon la revendication 1, caractérisé en ce que l'on fait réagir l'amine de formule générale:

$R - NH_2$

sur un dithioester de formule générale

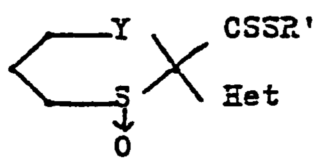

dont on a préalablement séparé les formes diastéréoisomères.

**Patentansprüche** für die VertragsstaatenBE, CH, DE, FR, GB, IT, LI, LU, NL, SE

1. Derivat des Thioformamids, dadurch gekennzeichnet, daß es der allgemeinen Formel

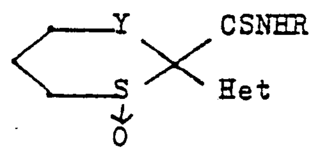

entspricht, worin R ein Wasserstoffatom oder einen Alkylrest mit 1 bis 4 Kohlenstoffatomen in gerader oder verzweigter Kette bedeutet, Het einen heterocyclischen Rest mit aromatischem Charakter, enthaltend ein Stickstoffatom, bedeutet, ausgewählt unter Pyridyl-3 oder Chinolyl-3,und Y eine Valenzbindung oder einen Methylenrest darstellt.

2. Verfahren zur Herstellung eines Produkts gemäß Anspruch 1, dadurch gekennzeichnet, daß man ein Amin der allgemeinen Formel

$R - NH_2$

worin R wie im Anspruch 1 definiert ist, auf einen Dithioester der allgemeinen Formel

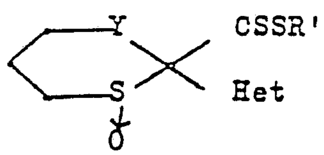

worin die Symbole Y und Het wie in Anspruch 1 definiert sind, und R' einen Alkylrest mit 1 bis 4 Kohlenstoffatomen in gerader oder verzweigter Kette oder einen Benzyl- oder Carboxymethylrest bedeutet, einwirken läßt, daß man dann das erhaltene Produkt isoliert und gegebenenfalls die stereoisomeren Formen trennt.

3. Verfahren gemäß Anspruch 2, dadurch gekennzeichnet, daß man das Amin der allgemeinen Formel

$R - NH_2$

auf einen Dithioester der allgemeinen Formel

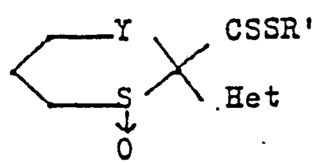

bei dem man vorher die diastereoisomeren Formen getrennt hat, einwirken läßt.

4. Arzneimittel, dadurch gekennzeichnet, daß es ein Produkt gemäß Anspruch 1 enthält, gegebenenfalls in Verbindung mit einem oder mehreren verträglichen und pharmazeutisch annehmbaren Verdünnungs- oder Hilfsmitteln.

**Patentansprüche** für den Vertragsstaat AT

1. Verfahren zur Herstellung eines neuen Thioformamidderivats der allgemeinen Formel:

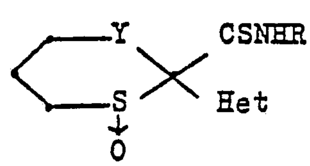

in welcher R für ein Wasserstoffatom oder einen geradkettigen oder verzweigten Alkylrest mit 1 bis 4 Kohlenstoffatomen steht, Het für einen hetrozyklischen Rest aromatischen Charakters mit einem oder zwei Stickstoffatomen ausgewählt unter 3-Pyridyl oder 3-Chinolyl steht und Y eine Valenzbindung oder einen Methylenrest bedeutet, dadurch gekennzeichnet, daß man ein Amin der allgemeinen Formel:

$R - NH_2$

in welcher R wie oben definiert ist, mit einem Dithioester der allgemeinen Formel:

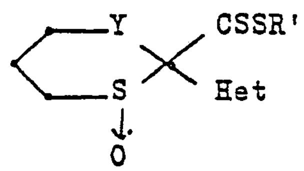

in welcher die Symbole Y und Het wie vorstehend definiert sind und R' für einen geradkettigen oder verzweigten Alkylrest mit 1 bis 4 Kohlenstoffatomen, einen Benzylrest oder einen Carboxymethylrest steht, umsetzt, worauf man das erhaltene Produkt isoliert und zutreffendenfalls die stereoisomeren Formen trennt.

2. Verfahren nach Anspruch 1, dadurch gekennzeichnet, daß man das Amin der allgemeinen Formel:

$R - NH_2$

mit einem Dithioester der allgemeinen Formel:

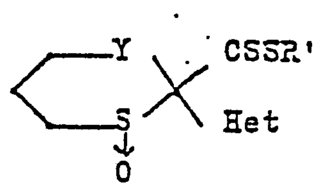

umsetzt, wobei man vorher die diastereoismeren Formen getrennt hat.

**Claims** for the Contracting States BE CH DE FR GB IT LI LU NL SE

1. A thioformamide derivative, characterised in that it corresponds to the general formula:

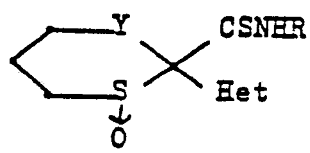

in which R denotes a hydrogen atom or an alkyl radical containing 1 to 4 carbon atoms as a straight or branched chain, Het denotes a heterocyclic radical of an aromatic nature containing a nitrogen atom, chosen from 3-pyridyl or 3-quinolyl, and Y denotes a valence bond or a methylene radical.

2. A process for the preparation of a product according to Claim 1, characterised in that an amine of general formula:

$R - NH_2$

in which R is defined as in Claim 1, is reacted with a dithioester of general formula:

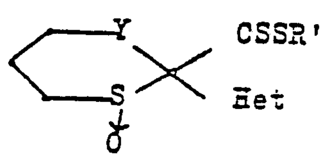

in which the symbols Y and Het are defined as in Claim 1 and R' denotes an alkyl radical containing 1 to 4 carbon atoms as a straight or branched chain or a benzyl or carboxymethyl radical, and then the product obtained is isolated and, where appropriate, the stereoisomeric forms are separated.

3. A process according to Claim 2, characterised in that the amine of general formula:

$R- NH_2$

is reacted with a dithioester of general formula:

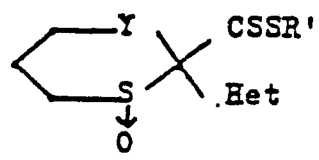

the diastereoisomeric forms of which have been separated beforehand.

4. A medicament characterised in that it contains a product according to Claim 1 optionally in combination with one or more compatible and pharmaceutically acceptable diluents or adjuvants.

**Claims** for the Contracting State AT

1. A process for the preparation of a new thioformamide derivative of general formula:

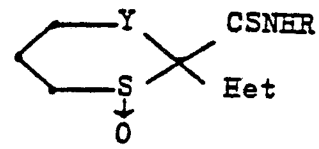

in which R denotes a hydrogen atom or an alkyl radical containing 1 to 4 carbon atoms as a straight or branched chain, Het denotes a heterocyclic radical of an aromatic nature containing one or two nitrogen atoms chosen from 3-pyridyl or 3-quinolyl, and Y denotes a valence bond or a methylene radical, characterised in that an amine of general formula:

$R - NH_2$

in which R is defined as above is reacted with a dithioester of general formula:

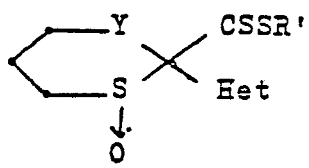

in which the symbols Y and Het are defined as above and R′ denotes an alkyl radical containing 1 to 4 carbon atoms as a straight or branched chain or a benzyl or carboxymethyl radical, and then the product obtained is isolated and, where appropriate, the stereoisomeric forms are separated.

2. A process according to Claim 1, characterised in that the amine of general formula:

$R - NH_2$

is reacted with a dithioester of general formula:

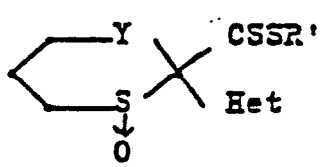

the diastereoisomeric forms of which have been separated beforehand.